# EUROPEAN PATENT APPLICATION

(11) **EP 3 560 546 A1**
(43) Date of publication of application: **30.10.2019**
(21) Application number: 18169633.7
(22) Date of filing: 26.04.2018
(51) Int. Cl.: A61M 37/00

(54) **MICRONEEDLE ARRAYS, AND METHODS AND SYSTEMS OF PRODUCING MICRONEEDLE ARRAYS HAVING A BRANCHED MATERIAL**

(71) Applicant: SABIC Global Technologies B.V., 4612 PX Bergen op Zoom (NL)
(72) Inventor: RAMAKRISHNAN, Vaidyanath, 4612 PX Bergen op Zoom (NL)
(74) Representative: J A Kemp LLP

(57) **Abstract**

This disclosure describes an array of microneedles formed from a polymer composition that includes a polymer and an additive, such as a hyperbranched polymer.

## Description

### FIELD OF INVENTION

The present disclosure relates generally to microneedle arrays, and, but not by way of limitation, to methods and systems for producing, such as microneedles formed from a polymer composition that includes a hyperbranched polymer as an additive.

### BACKGROUND

Health and wellness monitoring of a patient often requires sampling fluid (e.g., blood or interstitial fluid). For example, individuals with diabetes often use a lancing device to puncture tissue and draw blood provided to a test device, such as a blood glucose meter. As another example, a syringe is used to acquire fluid from a patient. Depending on an individual's health, multiple fluid samples may be acquired per day and, in severe situations, continuous sampling and monitoring may be warranted. Frequent or continuous sampling and monitoring is often painful, uncomfortable (e.g., causes skin sensitivity), and inconvenient.

A microneedle array has potential to provide a pain-free or reduced-pain alternative to a syringe for sampling fluid from a patient for testing (e.g., blood glucose level, insulin level, medication level, etc.). Microneedles can be virtually painless because they do not penetrate deep enough to contact nerves and are typically limited to penetration of the outermost layer of the skin, unlike traditional syringes and hypodermic needles. Additionally, a less shallow penetration may also reduce the risk of infection or injury. Microneedles may also facilitate delivery of a more precise dosage of a therapeutic which enables the use of lower doses in treatments. Other advantages of microneedles for drug delivery include the simplified logistics (absence of required cold chain), ability for patient self-administration (no need for doctor, nurse, reduction of people transport). As used in this disclosure, a "hollow" microneedle is a microneedle with an internal channel extending through at least a portion of the length of the microneedle and opening through at least one outer surface of the microneedle to permit fluid communication through the microneedle. In contrast, a "solid" microneedle is a microneedle that does not include a channel extending through an exterior surface of the microneedle, such that fluid communication is not permitted through the microneedle.

While there are a number of benefits to the use of microneedles, manufacturing of microneedle arrays has met many challenges to produce microneedle arrays at sufficient scale and efficiency such that they are cost effective. For example, given the relatively small sizes of microneedles and features of the microneedles, expensive production processes are needed. One approach that has been used to manufacture microneedles is injection molding. In such injection molding efforts, polymer has been melted and forced to flow into a mold that includes a plurality cavities or "negative" (female) molds each defining a microneedle. Stated another way, the mold defines a plurality of "inverse" microneedles. Injection molding thin and small parts such as microneedles can be a be quite challenging given the high shear rates. To illustrate, to have proper transcription of mold texture and shape to the molded part of a microneedle array, high flow may be necessary, especially having low viscosity at extremely high shear rates. Furthermore, good release from the production mold is important to reduce cycle time to improve the cost efficiency. The needles formed therefrom should exhibit good strength to prevent breaking of the microneedle during usage.

Conventional techniques to improve followability include using flow promoters which primarily act by reducing the glass transition temperature. Additionally, conventional techniques also use mold release agents to decrease the glassy transition temperature at the surface. However, use of such materials (e.g., flow promotors and/or mold release agents) can result in leaching or poor dispersion which lead to inconsistent performance. Thus, manufacturing of microneedle arrays faces challenges of identifying one or more techniques and/or one or more materials to manufacture microneedles that enable mold reproduction of thing parts and/or enable release of microneedles from a mold without damage.

### SUMMARY

The present disclosure describes a microneedle array, and methods and systems for producing the microneedle array. As described herein, the microneedle array may be formed by injection molding a polymer composite into a set of one or more molds defining a microneedle (e.g., a microneedle array). The polymer composite includes a polymer and an additive, such as a hyperbranched polymer. The hyperbranched polymer contributes to the polymer composition having a lower viscosity and/or a lower glass transition temperature than the polymer. Additionally, during injection of the polymer composite into the set of one or more molds, the hyperbranched polymer migrates to a surface of the polymer composition within the cavities of the mold. During removal of the plurality of microneedles from the mold, the hyperbranched polymer operates as a mold release agent. A microneedle array from the polymer composite may include microneedles has one or more characteristics selected from the group of characteristics consisting of: a tip having a radius of curvature of less than or equal to 25 micrometers (µm); a height extending from the needle base to a distal end of the microneedle and that is greater than or equal to 250 micrometers (µm); and an aspect ratio of the height to a transverse dimension of the needle base of greater than or equal to 1.

The polymer composite (including the polymer and the hyperbranched polymer) may advantageously lower a viscosity of the polymer composite as compared to the polymer. Additionally, or alternatively, the polymer composite (including the polymer and the hyperbranched polymer) may advantageously lower shear stress, which can otherwise contribute to processing related instabilities. The polymer composite may fill a mold cavity having a high aspect ratio thereby enabling mold reproduction, filling very thin parts and creating good replication of the needle tip. Additionally, or alternatively, the polymer composite may reduce or prevent processing instabilities (and the effects of processing instabilities), such as deformations (e.g., sharkskin) and aesthetic defects. The polymer composite can also improve mechanical properties of microneedles, such as elongation at break, impact properties, etc., as compared to conventional microneedles. Additionally, or alternatively, during injection molding, the hyperbranched polymer may migrate to the surface of the polymer composite whereby the hyperbranched polymer acts as a mold release agent that promotes release of microneedles and reduces breakage of microneedles during release. Accordingly, the present disclosure overcomes the existing challenges of forming microneedles with both high aspect ratios for a sharp tip (e.g., good tip replication during manufacturing) and a combination of properties (e.g., mechanical and/or chemical) for different phases of microneedle formation, by hot mold injecting the polymer composite including a hyperbranched polymer.

Some embodiments of the present apparatuses (e.g., of a microneedle array) comprise: a polymer composition having a first side and an opposing second side, the polymer composition defining a plurality of microneedles on the first side, each microneedle having a distal end and a base between the distal end and the second side of the multilayer polymer, the base of each of the microneedles having a cross-sectional area larger than that of the respective distal end; where each of the microneedles has one or more characteristics selected from the group of characteristics consisting of: a tip having a radius of curvature of less than or equal to 25 micrometers (µm); a height extending from the needle base to a distal end of the microneedle and that is greater than or equal to 250 micrometers (µm); and an aspect ratio of the height to a transverse dimension of the needle base of greater than or equal to 1; and where the polymer composition comprises a polymer and an additive, the additive comprises a hyperbranched polymer. In some implementations of the embodiments of the present apparatuses, the hyperbranched polymer comprises a hyperbranched copolymer.

In some of the foregoing embodiments of the present apparatuses, the hyperbranched polymer comprises a hyperbranched ring polymer or a hyperbranched star polymer. Additionally, or alternatively, in some of the foregoing embodiments of the present apparatuses, the hyperbranched polymer is present in an amount of less than 10 % by weight of the polymer composition. For example, the hyperbranched polymer may be present in an amount within a range of 0.1% to 2 % by weight of the polymer composition.

In some of the foregoing embodiments of the present apparatuses, each of the microneedles comprises: a tip having a radius of curvature of less than or equal to 25 µm; a height extending from the needle base to a distal end of the microneedle and that is from 400 µm to 1,000 µm; and an aspect ratio of the height to a transverse dimension of the needle base of greater than or equal to 2. In some such embodiments, the radius of curvature is less than or equal to 15 µm, the base of each of the microneedles comprises: a primary maximum transverse dimension measured in a first direction; and a secondary maximum transverse dimension measured in a second direction that is perpendicular to the first direction; and the transverse dimension of the aspect ratio comprises the primary maximum transverse dimension or the secondary transverse dimension.

In some of the foregoing embodiments of the present apparatuses, the base of each of the microneedles has a primary maximum transverse dimension measured in a first direction, and each of the microneedles has a height extending from the needle base to the distal end of the microneedle that is from 1 to 5 times the primary maximum transverse dimension of the respective microneedle. In some such embodiments, each of the microneedles has a secondary maximum transverse dimension measured in a second direction that is perpendicular to the first direction, and the ratio of the primary maximum transverse dimension to the secondary maximum transverse dimension of the respective microneedle is from 1 to 5.

In some of the foregoing embodiments of the present apparatuses, the polymer is selected from the group consisting of polycarbonate (PC), polymethylmethacrylate (PMMA), liquid-crystal polymer (LCP), polyether ether ketone (PEEK), fluorinated ethylene propylene (FEP), polysulfone (PSU), polyethylenimine, polyetherimide, polyimide (PI), polycarbonate copolymer (PC COPO), cyclic olefin copolymer (COC), cyclo olefin polymer (COP), polyamide (PA), acrylonitrile butadiene styrene (ABS), and polyphenylene ether (PPE). Additionally, or alternatively, in some of the foregoing embodiments of the present apparatuses, the polymer composition has a lower viscosity than the polymer, the polymer composition has a lower glass transition temperature than the polymer, or both.

Some embodiments of the present methods (e.g., of manufacturing a microneedle array) comprise: injecting a polymer composition into a mold defining a plurality of cavities such that the polymer composition flows into the cavities until the polymer substantially fills the cavities to form a plurality of microneedles, each of the cavities extending from a base at the surface to a distal end within the tool to define a negative mold of a microneedle, the base of each of the cavities having a cross-sectional area larger than that of the respective distal end; and removing the plurality of microneedles from the mold; where each of the microneedles has one or more characteristics selected from the group of characteristics consisting of: a tip having a radius of curvature of less than or equal to 25 micrometers (µm); a height extending from the needle base to a distal end of the microneedle and that is greater than or equal to 250 micrometers (µm); and an aspect ratio of the height to a transverse dimension of the needle base of greater than or equal to 1; and where the polymer composition comprises a polymer and an additive, the additive comprises a hyperbranched polymer.

In some of the foregoing embodiments of the present methods, the present methods further comprise combining, at an extrusion device, the polymer and the additive to form the polymer composition; and providing, from the extrusion device to an injection device, the polymer composition for injection into the mold. In a particular embodiment, the hyperbranched polymer migrates to a surface of the polymer composition within the cavities of the mold. In some such implementations, during removal of the plurality of microneedles from the mold, the hyperbranched polymer operates as a mold release agent.

Some embodiments of the present apparatuses (e.g., of a microneedle array) comprise: a microneedle array formed by any of the foregoing embodiments of the present methods.

As used herein, various terminology is for the purpose of describing particular implementations only and is not intended to be limiting of implementations. For example, as used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any disclosed embodiment, the term "substantially" may be substituted with "within [a percentage] of' what is specified, where the percentage includes .1, 1, or 5 percent; and the term "approximately" may be substituted with "within 10 percent of' what is specified. The phrase "and/or" means and or or. To illustrate, A, B, and/or C includes: A alone, B alone, C alone, a combination of A and B, a combination of A and C, a combination of B and C, or a combination of A, B, and C. In other words, "and/or" operates as an inclusive or.

The terms "comprise" (and any form of comprise, such as "comprises" and "comprising"), "have" (and any form of have, such as "has" and "having"), and "include" (and any form of include, such as "includes" and "including"). As a result, an apparatus that "comprises," "has," or "includes" one or more elements possesses those one or more elements, but is not limited to possessing only those one or more elements. Likewise, a method that "comprises," "has," or "includes" one or more steps possesses those one or more steps, but is not limited to possessing only those one or more steps.

Any embodiment of any of the systems, methods, and article of manufacture can consist of or consist essentially of - rather than comprise/have/include - any of the described steps, elements, and/or features. Thus, in any of the claims, the term "consisting of' or "consisting essentially of' can be substituted for any of the open-ended linking verbs recited above, in order to change the scope of a given claim from what it would otherwise be using the open-ended linking verb. Additionally, the term "wherein" may be used interchangeably with "where."

Further, a device or system that is configured in a certain way is configured in at least that way, but it can also be configured in other ways than those specifically described. The feature or features of one embodiment may be applied to other embodiments, even though not described or illustrated, unless expressly prohibited by this disclosure or the nature of the embodiments.

Some details associated with the embodiments are described above, and others are described below. Other implementations, advantages, and features of the present disclosure will become apparent after review of the entire application, including the following sections: Brief Description of the Drawings, Detailed Description, and the Claims.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. The figures are drawn to scale (unless otherwise noted), meaning the sizes of the depicted elements are accurate relative to each other for at least the embodiment depicted in the figures. Views identified as schematics are not drawn to scale.
**FIG. 1A** is a perspective view of a first example of a microneedle array.
**FIG. 1B** is a diagram that that illustrates a cross-section view of the microneedle array of FIG. 1A.
**FIGS. 2A and 2B** are perspective and side views, respectively, of an example of a microneedle included in a microneedle array of FIG. 1A.
**FIG. 3** is a diagram that illustrates examples of microneedle arrays.
**FIG. 4** is a graph that illustrates the loss modulus of a representative polymer melt.
**FIG. 5A** is a diagram that illustrates an example a cross-sectional view of a set of molds for forming the microneedle array of FIG. 1A.
**FIG. 5B** is a diagram that illustrates an example of stages of an injection molding process to form the microneedle array of FIG. 1A using the set of molds of FIG. 5B.
**FIG. 6** is a perspective view of an example of a system for producing a microneedle array.
**FIG. 7** is a flowchart illustrating an example of a method of manufacturing a microneedle array.

### DETAILED DESCRIPTION OF ILLUSTRATIVE EMBODIMENTS

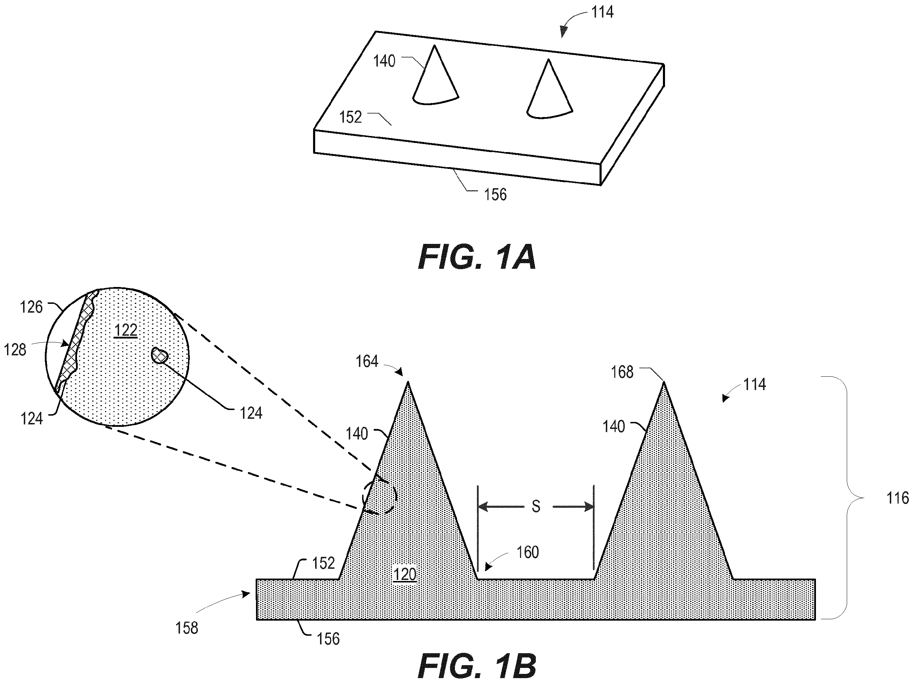

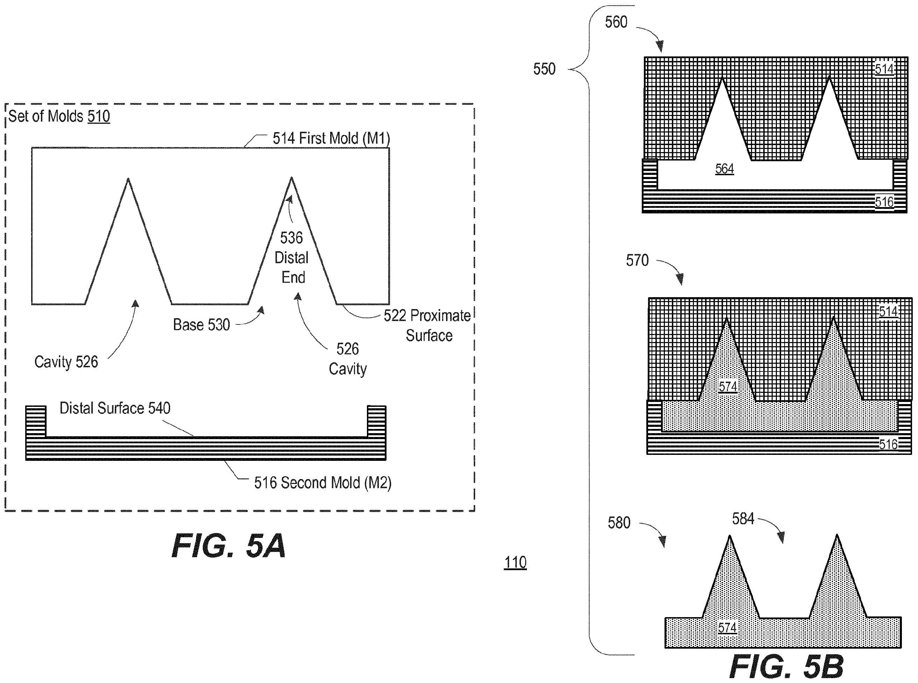

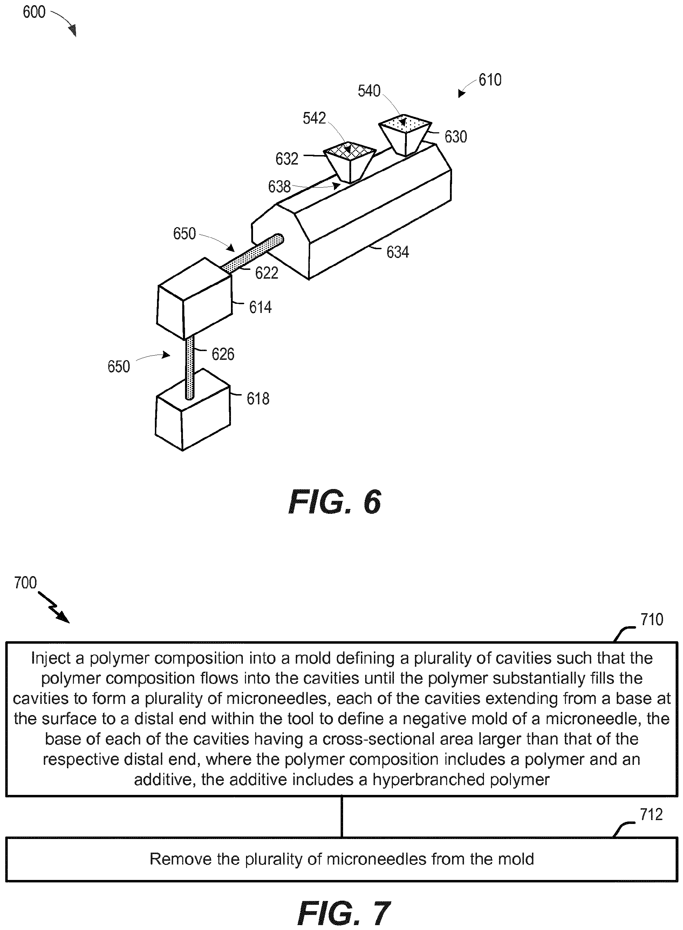

Referring to FIGS. 1A-B, FIG. 1A shows a perspective view of a first microneedle array 114 and FIG. 1B shows a cross-sectional view of microneedle array 114. Microneedle array 114 may include a polymer composite 120. Microneedle array 114 may be from using a process, such as an injection molding processes, as described herein at least with reference to FIGS. 5B and 6.

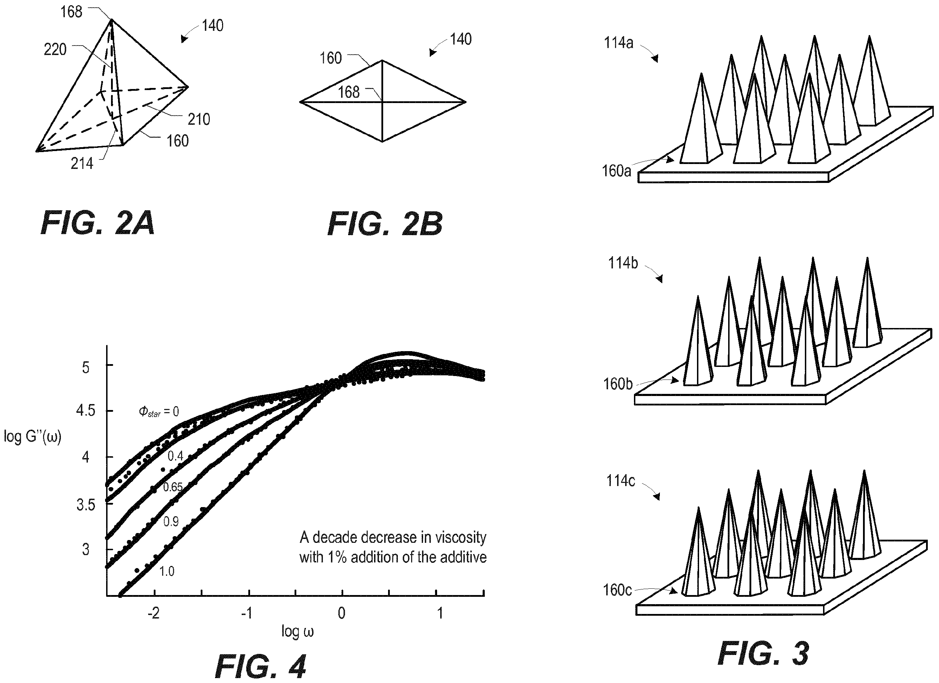

Microneedle array 114 includes a plurality of microneedles 140 extending from a layer of material (e.g., polymer composite 120) having a first side 152 (e.g., a first surface) and a second side 156 (e.g., a second surface). As shown, microneedles 140 extend from first side 152 of the layer. Although microneedle array 114 is illustrated as including two microneedles 140, in other implementations, microneedle array 114 can include a single microneedle or more than two microneedles, such as tens, hundreds, or thousands of microneedles. Additional examples (e.g., 114a-114c) of microneedle array 114 are described below with reference to FIG. 3.

Polymer composite 120 includes a polymer 122 and one or more additives, such as a hyperbranched polymer 124. Polymer 122 may include thermoplastic polymers, copolymers, and blends thereof. The polymer 122 may include a polyester, such as a semicrystalline polymer, polyethylene terephthalate (PET), polyethylene terephthalate glycol-modified (PETG), glycol-modified poly-cyclohexylenedimethylene terephthalate (PCTG), polycyclohexylenedimethylene terephthalate (PCT), isophthalic acid-modified polycyclohexylenedimethylene terephthalate (PCTA), and Tritan™ (a combination of dimethyl terephthalate, 1,4-cyclohexanedimethanol, and 2,2,4,4-tetramethyl-1,3-cyclobutanediol from Eastman Chemical). Additionally, or alternatively, the material may include a resin, such as Xylex™ (a combination of PC and an amorphous polyester), polybutylene terephthalate (PBT) (e.g., a resin from the Valox™ line of PBT), and/or a PET resin available from SABIC™. Additionally, or alternatively, the material may include liquid-crystal polymer (LCP), polyether ether ketone (PEEK), fluorinated ethylene propylene (FEP), polysulfone (PSU), polyethylenimine, polyetherimide, polyimide (PI), polymethylmethacrylate (PMMA), polycarbonate (PC), polycarbonate copolymer (PC COPO), cyclic olefin copolymer (COC), cyclo olefin polymer (COP), polyamide (PA), acrylonitrile butadiene styrene (ABS), polyphenylene ether (PPE), or a combination thereof.

The term "polycarbonate" (or "polycarbonates"), as used herein, includes copolycarbonates, homopolycarbonates and (co)polyester carbonates. The term polycarbonate can be further defined as compositions have repeating structural units of the formula (1): 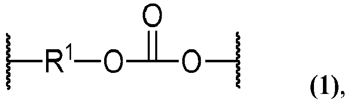 in which at least 60 percent of the total number of R1 groups are aromatic organic radicals and the balance thereof are aliphatic, alicyclic, or aromatic radicals. In a further aspect, each R1 is an aromatic organic radical and, more preferably, a radical of the formula (2):

-A1-Y1-A2- (2),

where each of A1 and A2 is a monocyclic divalent aryl radical and Y1 is a bridging radical having one or two atoms that separate A1 from A2. In various aspects, one atom separates A1 from A2. For example, radicals of this type include, but are not limited to, radicals such as -O-, -S-, -S(O)-, -S(O2)-, -C(O)-, methylene, cyclohexyl-methylene, 2-[2.2.1]-bicycloheptylidene, ethylidene, isopropylidene, neopentylidene, cyclohexylidene, cyclopentadecylidene, cyclododecylidene, and adamantylidene. The bridging radical Y1 may be a hydrocarbon group or a saturated hydrocarbon group, such as methylene, cyclohexylidene, or isopropylidene, as illustrative, non-limiting examples.

Hyperbranched polymers (e.g., 124) are three-dimensional, highly branched, oligomeric or polymeric molecules. For example, hyperbranched polymers can have highly branched, three-dimensional, tree-like structures, which can be characterized by having a "central unit" (e.g., a central monomer), one or more generations of branches, and an outermost generation of branches that terminate with end group functionalities. Hyperbranched polymers can be monodispersed, meaning that all molecules have the same structure, repeat units, and molecular weight. Monodispersed hyperbranched polymers generally have regular and highly symmetric branching. Such monodispersed polymers can be referred to in the art as "dendrimers" or "dendritic" polymers.

Hyperbranched polymers can also be polydispersed, meaning that the number of repeat units is not the same in all molecules. This results in a distribution of molecular weight between the various individual polyomeric chains within the composition. Furthermore, the polydispersed hyperbranched polymers have structures with branching that is neither regular nor highly symmetric, and may include linear segments. Polydispersed hyperbranched polymers are sometimes referred to in the art as simply "hyperbranched" polymers. However, for the purposes of this specification, the general term "hyperbranched" shall refer to both polydispersed and monodispersed hyperbranched polymers and the qualifier "polydispersed or "monodispersed" will be used when a specific type of hyperbranched polymer is intended to be referenced. Hyperbranched polymers (e.g., hyperbranched additives) may include polycarbonate (PC), polyolefins, polystyrene (PS), polyesters, polyether imides, polymethylmethacrylate (PMMA), siloxane (e.g., polyorthosiloxane (POSS), polysolxanes, etc.), or the like, as illustrative, non-limiting examples.

In some implementations, the hyperbranched polymer includes a hyperbranched copolymer. Additionally, or alternatively, the hyperbranched polymer may include a hyperbranched ring polymer or a hyperbranched star polymer. The amount of hyperbranched polymer 124 in polymer composition may be less than 10 % by weight of the polymer composition 120. For example, the amount of hyperbranched polymer 124 be in the range of from 0.1 to 10% by weight of the polymer composition 120. As another example, the hyperbranched polymer 124 may be present in an amount within a range of 0.1% to 2 % by weight of the polymer composition 120.

In some implementations, hyperbranched polymers can be characterized as: 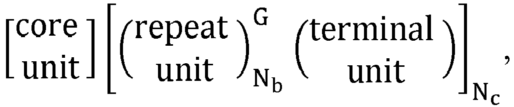 where a central core unit and some number of branches (N_{c}) attached to the core. Within each of the N_{c} branches attached to the core there are one or more sub-branches leading to terminal units. This is represented in the general formula by the terms G, which represents the number of generations of hyperbranch synthesis and N_{b} which is the branch multiplicity of the repeat unit.

Hyperbranched polymers may include a polymeric backbone material with terminal groups. For example, backbone groups may include polyesters, polysiloxanes, polyolefins, polystyrene and polyamides. The terminal unit may include or be related to one or more properties to be exhibited by the hyperbranched polymer. For example, the terminal unit can be selected from a group including hydroxy groups, alkyl groups, epoxy groups, silanol, acrylate and methlacrylate esters, trialkoxysilane, unsaturated fatty esters, aliphatic and aromatic carboxylic acid aesters and tertiary amines. In some implementations, the terminal unit(s) can be selected to achieve the surface properties which are desired in the final molded article, to obtain a particular melt flow index (MFI) of the polymer composition 120, or both.

The hyperbranched polymer may migrate to a surface of the microneedle array 114 during a molding process (e.g., an injection molding process). For example, referring to detail 126 of microneedle 140, polymer composition 120 includes polymer 124 and hyperbranched polymer 124, which is positioned along (and defines) a surface 128 of microneedle 140. Hyperbranched polymer 124 may migrate due to rheological differences between hyperbranched polymer 124 and polymer 122. To illustrate, when polymer composition 120 is subjected to a non-uniform deformation flow field (e.g., of a microneedle die), the polymer 120 (because of its chains that are substantially linear in shape) migrate away from regions of higher deformation. Conversely, the hyperbranched polymer 124, which typically have a non-linear shape, migrate towards regions of higher deformation. For example, hyperbranched polymer 124, due to its extremely high mobility (diffusion coefficient), small size and low viscosity move into regions of higher deformation which has been vacated by the polymer 122.

During a molding process to form microneedle 114, polymer composite 120 is formed and injected into a mold cavity. When the molten polymer composite 120 is injected into the mold cavity, the molten polymer composite 120 experiences large elongational gradients near the advancing flow front and large shear rates and rate gradients near the mold wall, which results in migration of hyperbranched polymer 124 towards the walls of the mold. As polymer composite 120 (e.g., hyperbranched polymer 124) reaches the wall, it is cooled down rapidly, thereby producing a surface which has a higher concentration of hyperbranched polymer 124 than the bulk (of the microneedle array 114).

The polymer composite 120 may further include one or more additives intended to impart certain characteristics to microneedle array 114. The various additives may be incorporated into polymer composition 120, with the proviso that the additive(s) are selected so as to not significantly adversely affect the desired properties of polymer 120 (e.g., the additives have good compatibility with the polymer). As illustrative, non-limiting examples, the composite polymer 120 may include an impact modifier, flow modifier, antioxidant, thermal (e.g., heat stabilizer), light stabilizer, ultraviolet (UV) light stabilizer, UV absorbing additive, plasticizer, lubricant, antistatic agent, antimicrobial agent, colorant (e.g., a dye or pigment), surface effect additive, radiation stabilizer, or a combination thereof. The additives may include impact modifiers such as styrene-butadiene thermoplastic elastomers, fire-retardant additives, colorants, thermal stabilizers, antioxidants, antistatic agents and flow promoters. Such additives can be mixed at a suitable time during the mixing of the components for forming the polymer composite 120.

As an illustrative, non-limiting example, polymer composite 120 may include, in addition to hyperbranched polymer 124, a mold release agent to facilitate ejection of a formed microneedle array from the mold assembly. Examples of mold release agents include both aliphatic and aromatic carboxylic acids and their alkyl esters, such as stearic acid, behenic acid, pentaerythritol tetrastearate, glycerin tristearate, and ethylene glycol distearate, as illustrative, non-limiting examples. Mold release agents can also include polyolefins, such as high-density polyethylene, linear low-density polyethylene, low-density polyethylene, and similar polyolefin homopolymers and copolymers. Additionally, some compositions of mold release agents may use pentaerythritol tetrastearate, glycerol monostearate, a wax, or a poly alpha olefin. Mold release agents are typically present in the composition at 0.05 to 10 wt %, based on total weight of the composition, such as 0.1 to 5 wt %, 0.1 to 1 wt%, or 0.1 to 0.5 wt%. Some mold release agents 1 have high molecular weight, typically greater than or equal to 300, to prohibit loss of the release agent from the molten polymer mixture during melt processing.

The composition includes a hyperbranched polymer from about 0.01 % to about 5%, by weight of the composition. As used herein, the term "hyperbranched polymer(s)" is intended to refer to poylmer(s) or oligomer(s) that have highly branched, three-dimensional, tree-like structures, which can be characterized by having a "nucleus", one or more generations of branches, and an outermost generation of branches that terminate with end group functionalities. As used herein, "nucleus" refers to a central monomer from which branches extend.

Microneedle 140 includes a base 160 and a distal end 164. Base 160 of a particular microneedle has a cross-sectional area larger than that of the respective distal end 164 of the particular microneedle. A tip 168 is positioned at distal end 164 of microneedle 140. Sharpness of tip 168 may be expressed by a radius of curvature(s) of tip 168. In a particular implementation, sharpness of tip 168 is less than 10 µm. In another implementation, sharpness of tip 168 is less than 20 µm. Alternatively, sharpness of tip 168 may be greater than or equal to 20 µm. Although microneedle 140 is illustrated as having a single tip, in other implementations, microneedle 140 may include multiple tips, a blade, or a combination thereof.

Additional characteristics (e.g., dimensions) of microneedle 140 are described with reference to FIGS. 2A and 2B, which show perspective and side views, respectively, of microneedle 140. Referring to FIGS. 2A and 2B, microneedle 140 is shaped as a pyramid and has base 160 and tip 168. As shown, base 160 has a rhomboid cross-sectional shape with a primary maximum transverse dimension 210 and a secondary maximum transverse dimension 214 measured perpendicular to primary maximum transverse dimension 210. Maximum transverse dimension 210 is greater than or equal to a secondary maximum transverse dimension 214 and may range from 75 µm to 350 µm. In other implementations, maximum transverse dimension 210 may be less than or equal to 75 µm, or is greater than or equal to 350 µm.

Microneedle 140 also has a height 220 that is measured perpendicular to both of the primary and secondary maximum transverse dimensions (210 and 214) of base 160. Height 220 of microneedle 140 is from 200 µm to 1,100 µm (e.g., from 400 µm to 1,000 µm, or from 400 µm to 800 µm). In a particular implementation, height 220 is 250 µm. In other implementations, height 220 is less than 250 µm or is greater than 1,000 µm.

In some implementations, height 220 of microneedle 140 is from one (1) to five (5) times primary maximum transverse dimension 210 (e.g., a height 220 of 1,000 µm and a primary maximum transverse dimension 210 from 200 µm to 1,000 µm, inclusive of 200 µm and 1,000 µm). In some such implementations, height 220 is from two (2) to four (4) times primary maximum dimension 210 (e.g., a height of 1,000 µm and a primary maximum dimension 210 from 250 µm to 500 µm).

In some implementations, height 220 is from one (1) to five (5) times secondary maximum transverse dimension 214 (e.g., a height 220 of 1,000 µm and a secondary maximum transverse dimension 214 from 200 µm to 1,000 µm, inclusive of 200 µm and 1,000 µm). In some such implementations, height 220 is from two (2) to four (4) times secondary maximum dimension 214 (e.g., a height of 1,000 µm and a secondary maximum dimension 214 from 250 µm to 500 µm). In one particular example, primary maximum transverse dimension 210 is 170 µm, secondary maximum transverse dimension 214 is 120 µm, and height 220 is 250 µm.

As shown in FIGS. 2A and 2B, microneedle 140 has a rhomboid cross-sectional shaped base (e.g., 160) and has a pyramid shape defined by four planar surface. In other implementations, base 160 may have a different cross-sectional shape, such as ellipsoid, triangular, square, rectangular, hexagonal, octagonal, star, or other shape. For example, referring to FIG. 3 examples (114a-114c) of microneedle array 114 are shown in which a first microneedle array 114a includes a base 160a having a rectangular (e.g., square) cross-sectional shape, a second microneedle array 114b includes a base 160b having a hexagonal cross-sectional shape, and a third microneedle array 114c includes a base 160c having an octagonal cross-sectional shape. Additionally, or alternatively, in other implementations, microneedle 140 can have any suitable outer surface profile or shape. For example, outer surfaces may be curved or curvilinear (e.g., concave) to result in a relatively sharper tip 168, or one or more blades along the vertices along which the outer surfaces of the microneedle meet one another may be curved or curvilinear (e.g., concave). In yet further configurations, the present microneedles can have any suitable shape that permits the microneedle to puncture a patient's skin as contemplated by this disclosure. It is noted that, in some implementations, at least a portion of microneedles 140 may vary in size (e.g., primary maximum transverse dimension 210, secondary maximum transverse dimension, height 220, cross-sectional base shape, and/or profile) relative to each other. This variation in size creates a varying aspect ratio in the microneedle array 114.

Additionally, with reference to FIG. 1B, spacing or distance (S) between two adjacent or closest microneedles may be different than or the same as primary maximum transverse dimension 210. To illustrate, spacing (S) may be less than primary maximum transverse dimension 210. In a particular implementation, spacing (S) may be within a range from 75 µm to 2 millimeters (mm). In other implementations, spacing (S) is greater than 2 mm.

As shown in FIGS. 1B, 2A, and 2B, microneedle 140 is a "solid" microneedle that does not include a channel extending through the microneedle. In other implementations, microneedle 140 may be a "hollow" microneedle with an internal channel extending through at least a portion of the microneedle.

The shape of the present microneedles (e.g., 140) are not particularly limited, but certain considerations may guide selection and design of different shapes. For example, the shape of a mold cavity to form the present microneedles (e.g., 140) may impact the ability to manufacture molds. Additionally, the shape of the mold cavity may impact the ease with which a microneedle array (e.g., 110) can be separated from a tool or mold after the polymer solidifies (e.g., the ease or lack thereof with which the microneedles can be removed from the cavities). For example, draft angles in the mold greater than 0.5 degrees may facilitate removal of a molded microneedle array from a mold. Additionally, or alternatively, the shape of the microneedle can impact the ability of the microneedle to puncture a patient's skin. For example, a microneedle may be designed to be strong enough to pierce the patient's skin and, while a broader base may result in a stronger microneedle, the increased angles of the sides of such a microneedle (with a broader base) may cause relatively greater trauma to the patient's skin. Thus, the aspect ratios discussed in this disclosure may be selected to result in microneedles with tips that are sharp enough to puncture a patient's skin with relatively low force, while causing minimal disruption to the surface of the patient's skin (e.g., the stratum corneum).

In a particular implementation, microneedle array 114 includes polymer composition 120 having first side 152, such as an exposed surface, and an opposing second side 156. Polymer composition 120 includes polymer 122 and an additive. The additive includes hyperbranched polymer 124. The polymer composition 120 defines a plurality of microneedles 140 on the first side 152. Each microneedle 140 having a distal end 164 and a base 160 between the distal end 164 and the second side 156. Base 160 of each of the microneedles 160 has a cross-sectional area larger than that of the respective distal end 164. Each of the microneedles 140 has one or more characteristics selected from the group of characteristics consisting of: a tip 168 having a radius of curvature of less than or equal to 25 micrometers (µm), a height 220 extending from the needle base 160 to distal end 164 of the microneedle 140 and that is greater than or equal to 250 micrometers (µm), and an aspect ratio of the height 220 to a transverse dimension of the needle base 160 of greater than or equal to 1.

The microneedle array 114 includes (and is formed from) composite polymer 120 that enables proper transcription of mold texture and shape to the molded features of the microneedle array. Polymer composite 120 may include high flow at high shear conditions to allow good transcription of mold texture and excellent filling of the finest mold features. For example, the hyperbranched polymer 124 may advantageously lower a viscosity and/or the shear stress of the polymer composite 120 as compared to the polymer 122. Additionally, or alternatively, the hyperbranched polymer 124 enables high release to have efficient de-molding and reduced cooling and cycle time during molding. For example, the hyperbranched polymer may migrate to the surface of the polymer composite during injection molding, where the hyperbranched polymer acts as a mold release agent that promotes release of microneedles and reduces breakage of microneedles during release. Additionally, or alternatively, the polymer composite 120 may reduce or prevent processing instabilities (and the effects of processing instabilities), such as deformations (e.g., sharkskin) and aesthetic defects. Accordingly, the present disclosure overcomes the existing challenges of forming microneedles with both high aspect ratios for a sharp tip (e.g., good tip replication during manufacturing) and a combination of properties (e.g., mechanical and/or chemical) for different phases of microneedle formation, by hot mold injecting the polymer composite including a hyperbranched polymer.

Referring to FIG. 4, FIG. 4 shows a graph 400 that illustrates a general representation of the loss modulus (G", which is a measure of viscous response or the viscosity) with shear rate (or frequency, ω) of a polymer melt (i.e., above the glass transition). For example, graph 400 illustrates that the addition of hyperbranched (*Φₛₜₐᵣ*, volume fraction) polymers lowers the viscosity, with increasing the content of the hyperbranched additive. The viscosity drop (e.g., the decrease in the entanglement density) results in a lower viscosity at lower shear rates. The hyperbranched additive also increases the shear thinning (i.e., lowers the onset of shear thinning), and lowering the viscosity at shear rates relevant to injection molding.

As shown, graph 400 can be understood as an example of as a general representation for a particular mechanism (e.g., polymer melt) and a particular hyperbranched additive (e.g., *Φₛₜₐᵣ*). It is understood that other mechanisms is different in different classes and/or other hyperbranched additives (e.g,. *Φₛₜₐᵣ*) could results in a different graphs. To illustrate, although graph 400 shows viscosity decreases continually with increases the hyperbranched content, there are cases where there is an initially decrease in viscosity followed by an increase.

Referring to FIGS. 5A-5B, FIG. 5A shows a cross-sectional view of an example of a set of molds 510 for forming microneedle array 114, and FIG. 5B shows stages of a molding process 650 to form array 114 using set of molds 510. As used herein, a mold may also be referred to as a tool or die.

Referring to FIG. 5A, set of molds 510 includes a first mold (M1) 514 and a second mold (M2) 516 that is configured to be positioned with respect to first mold (M1) 514. First mold (M1) 514 includes a proximate surface 522 that corresponds to first side 152 of microneedle array 114. First mold (M1) defines multiple cavities 526 that correspond to microneedles 140. Each of the cavities extends from a corresponding base 530 (e.g., opening) at proximal surface 522 to a distal end 536 within first mold (M1) 514 (e.g., a first tool) to define a negative mold of a microneedle. Base 530 of each of the cavities 526 has a cross-sectional area larger than that of the respective distal end 536. In a particular implementation, a cross-sectional shape of base 530 of each of the cavities 526 includes a circle. In other implementations, the cross-sectional shape of base 530 includes a shape other than a circle.

Second mold (M2) 516 includes a distal surface 540 and is configured to be positioned with respect to first mold (M1) 514 to define a space (e.g., a cavity) between first mold 514 and second mold 516. Although each of proximate surface 522 and distal surface 540 are illustrated as planar, in other implementations, proximate surface 522 and/or distal surface 540 may have a curved surface. For example, each of proximate surface 522 and distal surface 540 may have a convex surface.

Machining to form first mold 514, second mold 516, or both, may be formed via mechanical structuring, via micro electro discharge machining, via laser percussion drilling, or according to LIGA structuring, for example. LIGA may refer to a process combining lithography, electroplating, and replication to form microstructures in steel, for example.

Referring to FIG. 5B, cross-sectional views of stages of a molding process 550 using a set of molds (e.g., 510) is shown. Set of molds includes first mold (M1) 514 and second mold (M2) 516.

Referring to FIG. 5B, FIG. 5B shows injection molding process 550 in which first mold (M1) 514 is coupled to second mold (M2) 516 at a first stage 560. Second mold (M2) 516 is coupled to first mold (M1) 514 to define a space 564 (e.g., a cavity) between first mold (M1) 514 and second mold (M2) 516. At a second stage 570, a polymer composite 574 has been injected into the space 564. The polymer composite 574 may include or correspond to polymer composite 120. At a third stage 580, second mold (M2) 516 has been decoupled (e.g., removed) from first mold (M1) 514 to reveal the microneedle array 584. Microneedle array 584 may include or correspond to microneedle 114, 114a-114c.

Thus, FIGS. 5A and 5B depicts set of molds 510 and injection molding process 550 using polymer composited (e.g., 120, 574) to form microneedle array 584, such as microneedle array 114, 114a-114c. The polymer composite may advantageously fill a mold cavity defining thin parts of the microneedle. Additionally, or alternatively, during injection molding, the hyperbranched polymer may migrate to the surface of the polymer composite whereby the hyperbranched polymer acts as a mold release agent that promotes release of microneedles and reduces breakage of microneedles during release.

Referring to FIG. 6, an example of a system 600 for producing a microneedle array (e.g., 114, 114a-114c, 584) is shown. System 600 is configured to use an extrusion process to form a polymer composition (e.g.,) and an injection molding process to form the microneedle array from the polymer composition, as described herein. System 600 includes an extruder 610, injector 614, and die 618 (e.g., a mold). Extruder 610 is coupled to injector 614 via one or more conduits 622, such as one or more tubes. Injector 614 is coupled to die 618 via one or more conduits 626, such as one or more tubes.

Extruder 610 includes one more hoppers, such as a first hopper 630 and a second hopper 632, and a barrel 634 coupled to the one or more hoppers. For example, barrel 634 may be coupled to a hopper via a via a feed throat 638. Each hopper 530 is configured to receive material (e.g., pellets, granules, flakes, and/or powders) that is provided (e.g., gravity fed or force fed) from the hopper to barrel 634 via a corresponding feed throat 638. As shown, first hopper 630 has received a first material 640 and second hopper 632 has received a second material 642 (e.g., a material of third layer 410), and a third hopper has received a third material 544 (e.g., PC). First material 640 includes a polymer, such as polymer 122, and second material 642 includes an additive, such as hyperbranched polymer 124. Although described as being provided to separate hoppers, in other implementations, first and second materials 640, 642 may be provided the same hopper.

In some implementations, another material can be combined with first and second materials 640, 642 in the extruder 10. For example, the other material may be received by the extruder 610 via the one or more hoppers. The other material can include another additive, such as an impact modifier, flow modifier, antioxidant, heat stabilizer, light stabilizer, ultraviolet (UV) light stabilizer, UV absorbing additive, plasticizer, lubricant, antistatic agent, antimicrobial agent, colorant (e.g., a dye or pigment), surface effect additive, radiation stabilizer, or a combination thereof, as illustrative, non-limiting examples.

Each hopper provides its material into barrel 534 where the materials are combined to form a polymer composite 650. For example, the materials are gradually melted in barrel 534 by the mechanical energy (e.g., pressure) generated by turning screws, by heaters arranged along barrel 534, or both. The molten materials are mixed together (e.g., blended) to form polymer composite 650. Polymer composite 650 may include or correspond to polymer composite 120, 574. Polymer composite 650 is provided from barrel 534 via conduit 622 to injector 614. Injector 614 injects polymer composite 650 into die 618 via conduit 618. Polymer composition flows into the cavities until the polymer composition substantially fills the cavities to form a plurality of microneedles, such as a microneedle array (e.g., 114, 114a-114c, 584).

As shown, polymer composite 650 is provided from extruder 610 to die 618 via injector 614. In other implementations, system 600 may not include injector and extruder 610 may provide polymer composite 650 to die 618 via one or more conduits. Although polymer composite 650 has been described in system 600 using an extrusion process, in other implementations, polymer composite 650 may be formed by another process and provided to injector 614 for injection into die 618.

As described with reference to FIG. 6, system 600 is configured to form microneedle array. The microneedle array includes a polymer composite (including the polymer and the hyperbranched polymer) that may advantageously fill a mold cavity of the set of molds (e.g., die) thereby enabling mold reproduction, filling very thin parts and creating good replication of the needle tip. Additionally, or alternatively, during injection molding, the hyperbranched polymer may migrate to the surface of the polymer composite whereby the hyperbranched polymer acts as a mold release agent that promotes release of microneedles and reduces breakage of microneedles during release.

Referring to FIG. 7, an example of a method of manufacturing a microneedle array is shown. Method 700 may be performed by a manufacturing device or system, such as system 600. The microneedle array includes a plurality of microneedles and may include or correspond to microneedle array 114, 114a-114c, 584, as described herein.

Method 700 includes injecting a polymer composition into a mold defining a plurality of cavities such that the polymer composition flows into the cavities until the polymer composition substantially fills the cavities to form a plurality of microneedles, at 710. The polymer composition includes a polymer (e.g., 120, 574, 650) and an additive. The additive includes a hyperbranched polymer, such as hyperbranched polymer 124. Each of the cavities extending from a base at the surface to a distal end within the tool to define a negative mold of a microneedle. The base of each of the cavities having a cross-sectional area larger than that of the respective distal end. For example, the mold may include or correspond to set of molds 510 or die 618. Method 700 also includes removing the plurality of microneedles from the mold, at 812. The plurality of microneedles may be included in a microneedle array, such microneedle array 114, 1141-114c, 584. In some implementations, during removal of the plurality of microneedles from the mold, the hyperbranched polymer operates as a mold release agent.

In some implementations, the hyperbranched polymer (of the polymer composition injected into the mold) migrates to a surface of the polymer composition within the cavities of the mold. Method 700 may also include combining, at an extrusion device, the polymer and the additive to form the polymer composition. For example, the extrusion device may include or correspond to extruder 610. In some such implementations, method 700 also includes providing, from the extrusion device to an injection device, the polymer composition for injection into the mold. For example, the injection device may include or correspond to injector 614. In other implementations, the extrusion device may inject the polymer composition into the mold.

One product or article of manufacture that can be formed from method 700 includes a microneedle array including a polymer composition having a first side and an opposing second side, the polymer composition defining a plurality of microneedles on the first side, each microneedle having a distal end and a base between the distal end and the second side of the multilayer polymer. The polymer composition includes a polymer and an additive. The additive includes a hyperbranched polymer. The base of each of the microneedles having a cross-sectional area larger than that of the respective distal end. In some implementations, each of the microneedles has one or more characteristics selected from the group of characteristics consisting of: a tip having a radius of curvature of less than or equal to 25 micrometers (µm), a height extending from the needle base to a distal end of the microneedle and that is greater than or equal to 250 micrometers (µm), and an aspect ratio of the height to a transverse dimension of the needle base of greater than or equal to 1.

Thus, method 700 describes manufacturing of an array of microneedles, such as microneedle array 114, 114a-114c, 584. Method 700 advantageously enables polymer composite (including the polymer and the hyperbranched polymer) to fill a mold cavity of the die. For example, method 700 thereby enables polymer composite to fill very thin parts and create good replication of the needle tip. To further illustrate, the hyperbranched polymer may advantageously lower a viscosity and/or the shear stress of the polymer composite as compared to the polymer. Additionally, or alternatively, the hyperbranched polymer may migrate to the surface of the polymer composite during the injection process whereby the hyperbranched polymer acts as a mold release agent that promotes release of microneedles and reduces breakage of microneedles during release. Additionally, or alternatively, the polymer composite may reduce or prevent processing instabilities (and the effects of processing instabilities), such as deformations (e.g., sharkskin) and aesthetic defects. For example, polymer composite may include high flow at high shear conditions to allow good transcription of mold texture and excellent filling of the finest mold features.

The above specification and examples provide a complete description of the structure and use of illustrative embodiments. Although certain embodiments have been described above with a certain degree of particularity, or with reference to one or more individual embodiments, those skilled in the art could make numerous alterations to the disclosed embodiments without departing from the scope of this disclosure. As such, the various illustrative embodiments of the methods and systems are not intended to be limited to the particular forms disclosed. Rather, they include all modifications and alternatives falling within the scope of the claims, and embodiments other than the one shown may include some or all of the features of the depicted embodiments. For example, elements may be omitted or combined as a unitary structure, connections may be substituted, or both. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one embodiment or may relate to several embodiments. Accordingly, no single implementation described herein should be construed as limiting and implementations of the disclosure may be suitably combined without departing from the teachings of the disclosure.

The claims are not intended to include, and should not be interpreted to include, means-plus- or step-plus-function limitations, unless such a limitation is explicitly recited in a given claim using the phrase(s) "means for" or "step for," respectively.

## Claims

1. A microneedle array comprising:
a polymer composition having a first side and an opposing second side, the polymer composition defining a plurality of microneedles on the first side, each microneedle having a distal end and a base between the distal end and the second side of the multilayer polymer, the base of each of the microneedles having a cross-sectional area larger than that of the respective distal end;
where each of the microneedles has one or more characteristics selected from the group of characteristics consisting of:
a tip having a radius of curvature of less than or equal to 25 micrometers (µm);
a height extending from the needle base to a distal end of the microneedle and that is greater than or equal to 250 micrometers (µm); and
an aspect ratio of the height to a transverse dimension of the needle base of greater than or equal to 1; and
where the polymer composition comprises a polymer and an additive, the additive comprises a hyperbranched polymer.

2. The microneedle array of 1, where the hyperbranched polymer comprises a hyperbranched copolymer.

3. The microneedle array of any of claims 1-2, where the hyperbranched polymer comprises a hyperbranched ring polymer or a hyperbranched star polymer.

4. The microneedle array of any of claims 1-3, where the hyperbranched polymer is present in an amount of less than 10 % by weight of the polymer composition.

5. The microneedle array of claim 4, where the hyperbranched polymer is present in an amount within a range of 0.1% to 2 % by weight of the polymer composition.

6. The microneedle array of any of claim 1-5, where each of the microneedles comprises:
a tip having a radius of curvature of less than or equal to 25 µm;
a height extending from the needle base to a distal end of the microneedle and that is from 400 µm to 1,000 µm; and
an aspect ratio of the height to a transverse dimension of the needle base of greater than or equal to 2.

7. The microneedle array of any of claims 1-6, where:
the radius of curvature is less than or equal to 15 µm;
the base of each of the microneedles comprises:
a primary maximum transverse dimension measured in a first direction; and
a secondary maximum transverse dimension measured in a second direction that is perpendicular to the first direction; and
the transverse dimension of the aspect ratio comprises the primary maximum transverse dimension or the secondary transverse dimension.

8. The microneedle array of any of claims 1-7, where the base of each of the microneedles has a primary maximum transverse dimension measured in a first direction, and each of the microneedles has a height extending from the needle base to the distal end of the microneedle that is from 1 to 5 times the primary maximum transverse dimension of the respective microneedle.

9. The microneedle array of claim 8, where each of the microneedles has a secondary maximum transverse dimension measured in a second direction that is perpendicular to the first direction, and the ratio of the primary maximum transverse dimension to the secondary maximum transverse dimension of the respective microneedle is from 1 to 5.

10. The microneedle array of any of claims 1-9, where the polymer is selected from the group consisting of polycarbonate (PC), polymethylmethacrylate (PMMA), liquid-crystal polymer (LCP), polyether ether ketone (PEEK), fluorinated ethylene propylene (FEP), polysulfone (PSU), polyethylenimine, polyetherimide, polyimide (PI), polycarbonate copolymer (PC COPO), cyclic olefin copolymer (COC), cyclo olefin polymer (COP), polyamide (PA), acrylonitrile butadiene styrene (ABS), and polyphenylene ether (PPE).

11. The microneedle array of any of claims 1-10, where the polymer composition has a lower viscosity than the polymer, the polymer composition has a lower glass transition temperature than the polymer, or both.

12. A method of manufacturing a microneedle array, the method comprising:
injecting a polymer composition into a mold defining a plurality of cavities such that the polymer composition flows into the cavities until the polymer substantially fills the cavities to form a plurality of microneedles, each of the cavities extending from a base at the surface to a distal end within the tool to define a negative mold of a microneedle, the base of each of the cavities having a cross-sectional area larger than that of the respective distal end; and
removing the plurality of microneedles from the mold;
where each of the microneedles has one or more characteristics selected from the group of characteristics consisting of:
a tip having a radius of curvature of less than or equal to 25 micrometers (µm);
a height extending from the needle base to a distal end of the microneedle and that is greater than or equal to 250 micrometers (µm); and
an aspect ratio of the height to a transverse dimension of the needle base of greater than or equal to 1; and
where the polymer composition comprises a polymer and an additive, the additive comprises a hyperbranched polymer.

13. The method of claim 12, further comprising:
combining, at an extrusion device, the polymer and the additive to form the polymer composition; and
providing, from the extrusion device to an injection device, the polymer composition for injection into the mold.

14. The method of claim 12, where the hyperbranched polymer migrates to a surface of the polymer composition within the cavities of the mold, and where, during removal of the plurality of microneedles from the mold, the hyperbranched polymer operates as a mold release agent.

15. A microneedle array formed by the method of any of claims 12-14.
